# EUROPEAN PATENT SPECIFICATION

(11) **EP 3 846 901 B1**
(45) Date of publication and mention of the grant of the patent: **09.07.2025**
(21) Application number: 18762333.5
(22) Date of filing: 07.09.2018
(51) Int. Cl.: A61P 3/10, A61P 1/14, A61K 31/732, A61K 36/00

(54) **PREBIOTIC FOR TREATING DISORDERS ASSOCIATED WITH DISTURBED COMPOSITION OR FUNCTIONALITY OF THE INTESTINAL MICROBIOME**
PRÄBIOTIKUM ZUR BEHANDLUNG VON ERKRANKUNGEN IM ZUSAMMENHANG MIT GESTÖRTER ZUSAMMENSETZUNG ODER FUNKTIONALITÄT DES INTESTINALEN MIKROBIOMS
PRÉBIOTIQUE POUR LE TRAITEMENT DE TROUBLES ASSOCIÉS À UNE COMPOSITION OU À UNE FONCTIONNALITÉ PERTURBÉE DU MICROBIOME INTESTINAL

(43) Date of publication of application: 14.07.2021
(73) Proprietor: NutriLeads B.V., 6708 WH Wageningen (NL)
(72) Inventor: ALBERS, Ruud, 3235 KT Rockanje (NL); TZOUMAKI, Maria, 6708 WH Wageningen (NL)
(74) Representative: HGF
(86) International application number: PCT/EP2018/074127
(87) International publication number: WO 2020/048609

(56) References cited:
- WO-A1-2012/148277
- GÓMEZ BELÉN ET AL: "Prebiotic potential of pectins and pectic oligosaccharides derived from lemon peel wastes and sugar beet pulp: A comparative evaluation", JOURNAL OF FUNCTIONAL FOODS, vol. 20, 10 November 2015 (2015-11-10), pages 108 - 121, XP029360124, ISSN: 1756-4646, DOI: 10.1016/J.JFF.2015.10.029
- KIM HOON ET AL: "Effect of arabinoxylan- and rhamnogalacturonan I-rich polysaccharides isolated from young barley leaf on intestinal immunostimulatory activity", JOURNAL OF FUNCTIONAL FOODS, ELSEVIER BV, NL, vol. 35, 9 June 2017 (2017-06-09), pages 384 - 390, XP085116425, ISSN: 1756-4646, DOI: 10.1016/J.JFF.2017.05.052
- BABBAR ET AL: "Pectic oligosaccharides from agricultural by-products: production, characterization and health benefits", CRIT. REV. BIOTECH,, vol. 36, no. 4, 2 February 2015 (2015-02-02), pages 594 - 606, XP002778159

## Description

### TECHNICAL FIELD OF THE INVENTION

The present invention relates to prebiotic compositions for use in a method of therapeutic or prophylactic treatment of disorders associated with disturbed composition or functionality of the intestinal microbiome in a subject, the disorder being selected from overweight, obesitas, insulin-resistance and intestinal barrier dysfunction, said method comprising oral administration of a prebiotic composition to the subject, said prebiotic composition containing rhamnogalacturonan I (RG-I) polysaccharides originating from fruit, carrot, pea, chicory or sugar beet.

The invention further relates to a prebiotic composition and a synbiotic composition suitable for use in the aforementioned method of treatment.

### BACKGROUND OF THE INVENTION

Relation between the gut microbiota and human health is being increasingly recognised. It is now well established that a healthy gut microbiota is largely responsible for overall health of the host.

The human host provides a habitat and nutrition to a large and diverse ecosystem of microbial communities that play a crucial role in digestion, metabolism and modulation of immune function, and that have a significant impact beyond the gastrointestinal tract. Changes in the diversity and function of those communities are associated with far reaching consequences on host health and have been linked with a number of disorders, including functional bowel disorders, inflammatory bowel diseases and other immune mediated diseases (coeliac disease, allergies) and metabolic conditions (type 2 diabetes, NASH). Dysbiosis (also called dysbacteriosis) is a term for a microbial imbalance or maladaptation on or inside the body, such as an impaired microbiota. For example, bacterial communities occupying certain surface areas of a host such as the gut microbiota, skin microbiota or vaginal microbiota, can become modified/altered with normally dominating species underrepresented and normally outcompeted or contained species increasing to undesired levels. The collection of bacteria in such communities are collectively called the microbiota. Local microbiota together with other micro-organisms including yeast, fungus, viruses, and parasites residing in such niches are jointly called the microbiome. Dysbiosis is not limited to imbalances in the microbiota, but may involve other micro-organisms in the microbiome as well (e.g. viruses, archaea and fungi).

When a microbiome is well balanced, called normobiosis, the microorganisms occupying a specific niche form a more or less stable community that is well adapted to the local conditions, has the metabolic capabilities to live of the available substrate, effectively deals with stressors and modulation of substrate availability and has regulatory mechanisms in place that contribute to the meta-stability of the community and contributes to long-term health of its host.

Dysbiosis is most commonly studied as a condition in the gastrointestinal tract, but it can affect any body cavity, mucosal and skin surfaces populated by a microbial community.

Dysbiosis has been associated with illnesses of the host; intestinal dysbiosis has for instance been associated with inflammatory bowel disease, chronic fatigue syndrome, obesity, cancer, cardiometabolic conditions, insulin insensitivity, (pre) diabetes, bacterial vaginosis, and colitis.

Composition and stability of the microbiota are influenced by the genetic background of the host, by environmental conditions or stressors that include, for example, diet, lifestyle, use of medicaments - e.g. antibiotics -, and developmental stage (age) of the host. This translates into a permanent and complex interaction between the host and the main components of the local microbial ecosystem. These components include the microbiota, the host immune system, the local epithelial barrier and, in case of the gut, the enteric nervous system.

The neonate microbiota is establishing itself since birth onwards and is notably fluctuating during the first weeks and months of life to become close to the core adult microbiota around 3-4 years of age. This progressive microbial colonization of the gut is key for the education and maturation of the host immune and enteric nervous systems, gut barrier and function, and metabolic programming of the host, which has an impact on the short term and later in life health status and risk of diseases. The neonatal microbiota is influenced by the maternal diet and microbiota, by the delivery mode, by the infant nutrition (breast feeding or infant formula) and the surrounding environmental conditions.

In contrast, the healthy adult core microbiota is more stable in the sense that when perturbed by various stressors (e.g. use of antibiotics or medicaments) it returns close to its original composition in healthy subjects, which is designated as resilience of the microbiota. Loss of variability, absence or low abundance of beneficial microorganisms, and loss of resilience are all associated with diseases.

The microbiota of ageing or elderly subjects is diverging from that in the healthy adult population by changes in composition leading to less bacterial diversity, reduction of beneficial microorganisms and reduced resilience. All of these changes are associated to changes in the health status.

The microbiota is composed of a large variety of species that compete for space and resources/nutrients, but that can also feed each other with their respective fermentation products, thus leading to consortia of microorganisms that, in healthy status, live in symbiosis with the mammal host. A high microbial diversity within the intestinal microbiota is considered beneficial for the health of the host, as diversity renders the microbiota more resilient to factors that disturb the intestinal microbiota (e.g antibiotics, change in diet, invasion by new species). Also important are consortia of microbes, i.e. metastable combinations of different microorganisms that feed of each other's fermentation products and jointly form a more or less stable ecosystem that thrives in a particular niche using available substrate (e.g. from mucus, cell shedding and the diet).

Typical microbial species found on or in the body are mostly beneficial or harmless. Pathobionts or even pathogens are also part of a "normal" microbiota as long as they remain below a critical level. The mammal intestinal microbiota carries out a series of helpful and necessary functions, such as aiding in digestion, providing energy from food, providing specific (micro) nutrients to the host, producing key metabolites such as short chain fatty acids, and educating (neonates and infants) or maintaining (adults) the host's immune system. They also help protect the body from incoming pathogenic microbes or toxic compounds.

Microbial species also excrete many different types of waste byproducts. Using different waste removal mechanisms, under normal circumstances the body effectively manages these byproducts with little or no trouble. Unfortunately, oversized microbial populations and inappropriate dominance of microbial species, due to their increased numbers, excrete increased amounts of these byproducts. As the amount of microbial byproducts increases, the higher waste byproducts levels can overburden the body's waste removal mechanisms. An example of this is the formation of ammonia from protein fermentation which can be further fermented into compounds that are detrimental to the host.

It is the relative dominance or underrepresentation of particular microbial species, low diversity and/or disturbed production of microbial metabolites that causes many of the negative health symptoms observed in subjects suffering from dysbiosis.

Consumption of probiotics and/or prebiotics can have a favourable impact on the intestinal microbiota.

Probiotics are "live micro-organisms which, when administered in adequate amounts, confer a health benefit on the host" (definition of World Health Organization).

Prebiotics are nondigestible food ingredients that beneficially affect the host by selectively stimulating the growth and/or activity of one or a limited number of microbial species in a community.

Most known prebiotics are simple oligomers of identical sugars (such as fructose, galactose or arabinose) linked by glycosidic bonds. These stimulate the selective outgrowth of microbial species that have the metabolic capabilities to (rapidly) ferment these relatively simple substrates to produce beneficial metabolites such as short chain fatty acids. Typical side effects of the use of such easily fermentable substrates include intestinal discomfort, flatulence and regurgitation. These side effects are caused by the rapid fermentative production of gasses.

Pectin is a structural hetero polysaccharide that is present in the primary cell walls of terrestrial plants.

Pectic polysaccharides are a heterogeneous group of polysaccharides comprising varying amounts of the following polysaccharide components:
(i) homogalacturonan (HG),
(ii) xylogalacturonan (XG),
(iii) apiogalacturonan (AG)
(iv) rhamnogalacturonan-I (RG-I), and
(v) rhamnogalacturonan-II (RG-II).

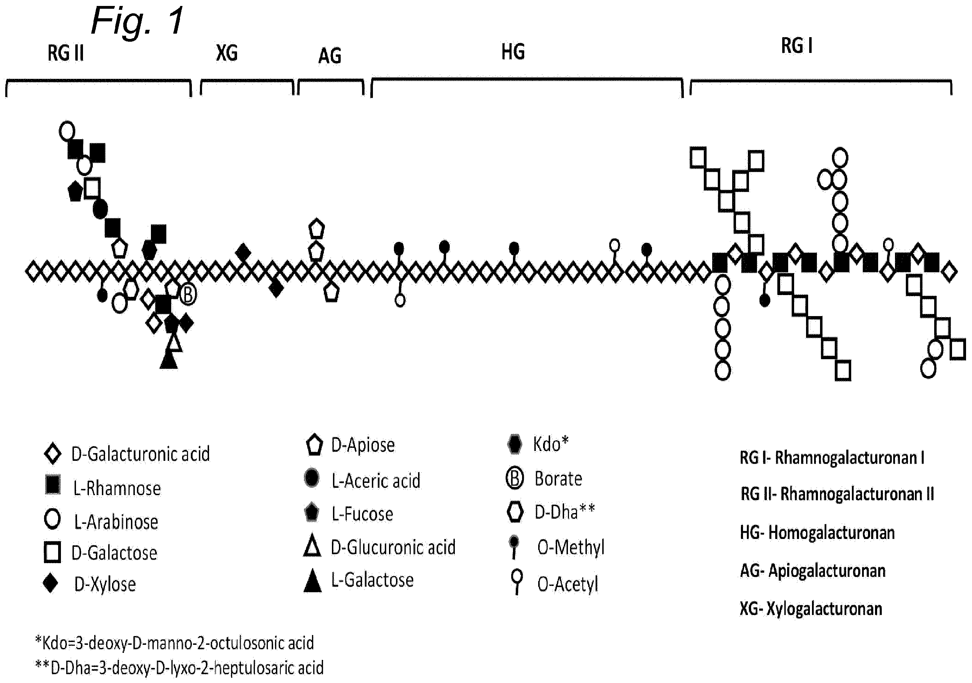

Figure 1 provides a schematic representation of the structure of pectic polysaccharides, including the aforementioned 4 polysaccharide components. It is noted that the polysaccharide components HG, XG and RG-II typically represent only a minor fraction of pectic polysaccharides.

The polysaccharide components HG, XG and RG-II each comprise a backbone that consists of a linear chain of α-(1-4)-linked D-galacturonic acid monosaccharide units.

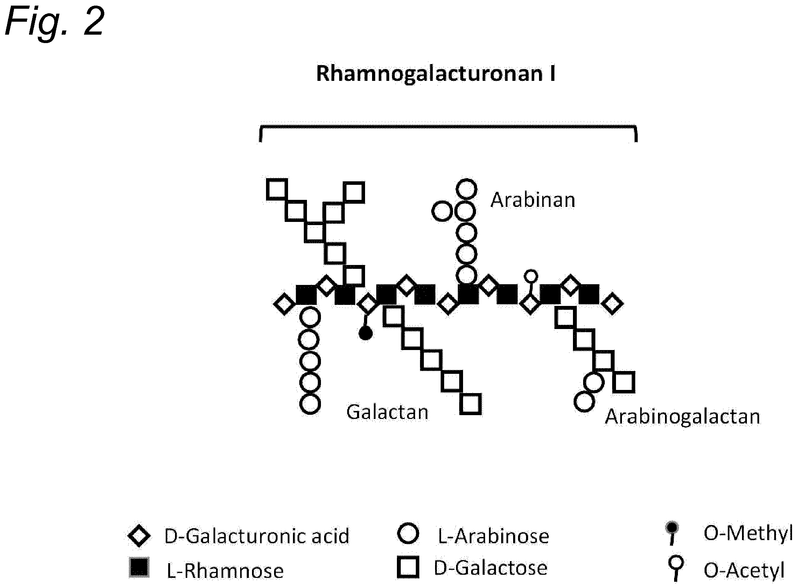

Only RG-I comprises a backbone that consists of a linear chain of the repeating disaccharide units: 4)-α-D-galacturonic acid-(1,2)-α-L-rhamnose-(1. A schematic representation of the structure of RG-I is shown in Figure 2.

Pectic polysaccharide composition and fine structure vary widely depending on the plant source and the extraction conditions applied. The homogalacturonan domain can have a length of up to about 100 consecutive D-GalA residues. The RG-I domain containing the side chains is usually called the 'ramified region' or 'hairy region', while the homogalacturonan domain (between two RG-I domains) is not typically substituted with oligosaccharides.

The GalA residues in RG-I are linked to the Rha residues via the 1 and 4 positions, while the Rha residue is linked to the GalA residue via the anomeric and 2-OH positions. In general about 20-80% of the Rha residues is branched at the 4-OH position (depending on the plant source and the method of isolation), with neutral and acidic side chains. These side chains consist mainly of Ara and Gal residues linked in various manners, constituting polymers known as arabinogalactan I (AG-I) and/or AG-II. AG I is composed of a beta-(1,4)-linked D-Gal backbone with substitutions at 3-OH of alpha-L-arabinosyl groups; the Gal backbone can have interspacing alpha(1,5)-L-Ara units. AG-II consists of highly ramified galactan with predominantly interior beta(1,3)-linked D-Gal with substitutions of short (1,6)-linked chains exteriorly. The latter has further attachments of (1,3)- and/or alpha(1,5)-linked L-Ara. The oligosaccharide side chains may be linear or branched, and some of these side chains may be terminated with alpha-L-fucosides, beta-D-glucuronides, and 4-O-methyl beta-D-glucuronyl residues.

Gómez et al. (Prebiotic potential of pectins and pectic oligosaccharides derived from lemon peel wastes and sugar beet pulp: A comparative evaluation, Journal of Functional Foods, Volume 20, January 2016, Pages 108-121) described the outcome of a study in which sugar beet pulp (SBP) and lemon peel wastes (LPW) were used to obtain two mixtures of pectic oligosaccharides (denoted as SBPOS and LPOS, respectively). The suitability of pectic oligosaccharides, pectins from SBP and LPW and commercial FOS for causing prebiotic effects were compared by *in vitro* fermentation and fluorescence *in situ* hybridization using human faecal inocula and eight different bacterial probes. The joint populations of bifidobacteria and lactobacilli increased from 19% up to 29%, 34% and 32% in cultures with LPOS, SBPOS and FOS, respectively. *Faecalibacterium* and *Roseburia* counts were also increased with all the substrates (especially with LPOS). The highest concentrations of organic acids were observed in media containing oligosaccharides. According to the authors, this work confirms that pectic oligosaccharides present better prebiotic properties than pectins, and similar or better than FOS.

Chatterjee, et al. (Effect of Fruit Pectin on Growth of Lactic Acid Bacteria, J Prob Health 2016, 4:2) report a study in which the effect of pectin extracted from different types of fruit waste (*Musa* sp. and *Citrus limetta* and rind of *Citrullus lanatus* and putrefied fruits of *Solanum lycopersicum* and *Psidium guajava*) on growth of Lactic Acid Bacteria (LAB) and bifidobacteria (*Lactobacillus casei, L. acidophilus* and *Bifidobacterium bifidum*) was tested. It was observed that pectin was able to enhance the growth of the bacteria and the titrable acidity considerably. The authors conclude that pectin that is extracted from fruit waste can be used to enhance the growth of lactobacilli and bifidobacteria.

Babbar, et al. (Pectic oligosaccharides from agricultural by-products: production, characterization and health benefits, Crit. Rev. Biotech. 2016; 36(4) 594-606) mention that pectin containing agricultural by-products are potential sources of a new class of prebiotics known as pectic oligosaccharides (POS). Controlled hydrolysis of pectin containing agricultural by-products like sugar beet, apple, olive and citrus by chemical, enzymatic and hydrothermal treatment can be used to produce oligo-galacturonides, galactooligosaccharides, rhamnogalacturonan-oligosaccharides, etc..

Hoon Kim et al. (Effect of arabinoxylan- and rhamnogalacturonan I-rich polysaccharides isolated from young barley leaf on intestinal immunostimulatory activity, Journal of Functional Foods, 2017; 35, 384-390) prepared four polysaccharide fractions from barley leaves and compared *in vitro* intestinal immunostimulatory activity. Among fractions, a high molecular weight fraction prepared by enzyme extraction (BLE-P), showed the potent in activating bone marrow cell proliferation through Peyer's patch (PP), and in stimulating cytokine production in *in vitro.* BLE-P was identified as a mixture of hemicellulosic glucuronoarabinoxylan and pectic rhamnogalacturonan I accounting for above 80%. Subsequently, BLE-P was orally administered to mice for 20 days to investigate the effect on the *in vivo* intestinal immunostimulatory activity. BLEP administration not only augmented the production of immunoglobulin A (IgA), but also increased the levels of IgA-related cytokines, such as transforming growth factor-β and interleukin-10.

US 2014/275233 describes a method for treating gastrointestinal dysbiosis in a subject, comprising orally administering to the subject an effective amount of a composition comprising isolated plant tissue having at least 0.25 mg glyceollin content per gram of plant tissue. Three very similar phytoalexins called glyceollin I, glyceollin II, and glyceolin III, are produced by soy when the plant is exposed to soil microorganisms, ultraviolet (UV) light or heavy metals.

WO 2011/069781 describes a polysaccharide that is capable of modulating immune response, said polysaccharide being obtained from plants of the species Camellia sinensis, wherein the backbone of the polysaccharide comprises alternating rhamnogalacturonan-I domains and alpha(1,4)-linked polygalacturonic acid or alpha(1,4)-linked oligogalacturonic acid domains, wherein the molar ratio of galacturonic acid residues to rhamnosyl residues in the backbone of the polysaccharide ranges from 2.5:1 to 1 :1, and wherein the polysaccharide has a molecular weight of at least 70 kDa.

WO 2012/148277 describes a preparation having a dry matter content of at least 20 wt.%, said preparation containing at least 50% by weight of dry matter of a mixture of pectic polysaccharides, including at least 20%, calculated by weight of the pectic polysaccharides, of rhamnogalacturonan-I pectins having a molecular weight of more than 40 kDa, said mixture of pectic polysaccharides being characterized by:
- a degree of methylation of the galacturonic acid residues of not more than 20%;
- a degree of acetylation of the galacturonic acid residues of not more 20%;
wherein the preparation does not form a gel when it is diluted with an aqueous solution of 50 mM ammoniumbicarbonate to a solids content of 2.5 wt.%. Also described is the use of this preparation as a medicament to modulate immune response.

### SUMMARY OF THE INVENTION

The invention is defined by the claims.

The inventors have unexpectedly discovered that disorders associated with disturbed composition or functionality of the intestinal microbiome, notably metabolic disorder or intestinal barrier dysfunction, may be treated therapeutically or prophylacatically by orally administering rhamnogalacturonan I (RG-I) polysaccharides originating from fruit, carrot, pea, chicory or sugar beet, said RG-I polysaccharides having a molecular weight in excess of 15 kDa and having a backbone comprising rhamnogalacturonan-I domains and optionally alpha(I,4)-linked homo-galacturonic acid domains, wherein the molar ratio of galacturonic acid residues to rhamnose residues in said backbone is within the range of 20:1 to 1:1. Although the inventors do not wish to be bound by theory, it is believed that the RG-I polysaccharides act as a prebiotic by promoting high microbial diversity within the intestinal microbiota, by stimulating growth or activity of beneficial bacteria (e.g. *Akkermansia muciniphila* and *Bifidobacterium spp.)* and/or by increasing the resilience of the intestinal microbiota against disturbances.

It has further been found that intestinal fermentation of the RG-I polysaccharides results in the formation of beneficial short chain fatty acids. Surprisingly, fermentative conversion of the RG-I polysaccharides to short chain fatty acids is accompanied by substantially less gas production than is observed for classical prebiotics such as e.g. inulin.

Accordingly one aspect of the present invention relates to a prebiotic composition for use in a method of therapeutic or prophylactic treatment of disorders associated with disturbed composition or functionality of the intestinal microbiome in a subject, the disorder being selected from overweight, obesitas, insulin-resistance and intestinal barrier dysfunction said use comprising oral administration of the prebiotic composition to the subject, wherein the composition contains at least 0.1% by weight of dry matter of RG-I polysaccharides originating from fruit, carrot, pea, chicory or sugar beet, said RG-I polysaccharides having a molecular weight in excess of 15 kDa and having a backbone consisting of galacturonic acid residues and rhamnose residues, said rhamnose residues being contained in alpha(1→4)-galacturonic-alpha(1→2)-rhamnose residues, wherein the molar ratio of galacturonic acid residues to rhamnose residues in the RG-I polysaccharides is within the range of 20:1 to 1:1.

The RG-I polysaccharides that are employed in accordance with the present invention may be isolated from fruit, carrot, pea, chicory or sugar beet by aqueous extraction, optionally combined with enzymatic treatment (using e.g. polygalacturonase).

Also disclosed but not part of the invention is a prebiotic composition comprising:
- at least 0.1% by weight of dry matter of the aforementioned RG-I polysaccharides; and
- at least 1% by weight of dry matter of one or more prebiotics selected from lactulose, inuline, fructooligosaccharides, galactooligosaccharides, milk oligosaccharides, guar gum, gum Arabic or any combinations thereof.

Yet another aspect of the invention relates to a synbiotic composition comprising:
- at least 0.1% by weight of dry matter of the RG-I polysaccharides; and
- 10⁴ to 10¹⁰ cfu/gram of *Akkermansia muciniphila* and/or 10⁶ to 10¹⁰ cfu/gram of *Bifidobacterium spp.*

### DETAILED DESCRIPTION OF THE INVENTION

One aspect of the invention relates to a prebiotic composition for use in a method of therapeutic or prophylactic treatment of disorders associated with disturbed composition or functionality of the intestinal microbiome in a subject, the disorder being selected from overweight, obesitas, insulin-resistance and intestinal barrier dysfunction said use comprising oral administration of the prebiotic composition to the subject, wherein the composition contains at least 0.1% by weight of dry matter of rhamnogalacturonan I (RG-I) polysaccharides originating from fruit, carrot, pea, chicory or sugar beet, said RG-I polysaccharides having a molecular weight in excess of 15 kDa and having a backbone consisting of galacturonic acid residues and rhamnose residues, said rhamnose residues being contained in alpha(1→4)-galacturonic-alpha(1→2)-rhamnose residues, wherein the molar ratio of galacturonic acid residues to rhamnose residues in the RG-I polysaccharides is within the range of 20:1 to 1:1.

The terminology "disorders associated with disturbed composition or functionality of the intestinal microbiome" as used herein encompasses intestinal dysbiotic condition (disturbed composition) and disorders associated with insufficient fermentative production of essential metabolites by the intestinal microbiome (disturbed functionality). Short chain fatty acids (acetate, propionate, and butyrate) are an example of such essential metabolites.

The term "intestinal dysbiotic condition" as used herein refers to a condition that adversely affects a subject's health and that is caused by a significant deviation from a balanced intestinal microbiome (normobiosis).

The term "branched polysaccharides" as used herein refers to a polysaccharides comprising a linear backbone chain of monosaccharide units bound together by glycosidic linkages, wherein at least one of the monosaccharide units within the backbone chain carries a sidechain of one or more glycosidically linked monosaccharide units.

The terms "backbone chain" and "backbone" are synonyms.

The term "pectic polysaccharides" as used herein refers to optionally branched polysaccharides having a molecular weight in excess of 15 kDa and comprising a backbone that consists of galacturonic acid residues and rhamnose residues, said rhamnose residues being contained in alpha(1→4)-galacturonic-alpha(1→2)-rhamnose residues.

The term "stretch" as used herein refers to a sequence of two or more glycosidically linked monosaccharide within the backbone of a polysaccharide, excluding any sidechains that are attached thereto.

The term "domain" as used herein refers to a stretch plus any sidechains that are attached to said stretch.

The term "rhamnogalacturonan-I stretch" or "RG-I stretch" refers to a stretch consisting of galacturonic acid (GalA) and rhamnose (Rha) pairs, wherein the GalA residues are linked to the Rha residues via the 1 and 4 positions, while the Rha residues are linked to the GalA residue via the anomeric and 2-OH positions, i.e. alternating alpha(1→4)-galacturonic-alpha(1→2)-rhamnose residues. The carboxyl groups of the galacturonic acid residues within the RG-I stretches may be esterified. Esterified galacturonic acid may occur in the form of the methyl ester or acetyl ester.

The RG-I domain can comprise side chains such as, for example galactan, arabinan and arabinogalactan side chains.

The term "rhamnogalacturonan-I polysaccharide" or "RG-I polysaccharide" refers to an optionally branched pectic polysaccharide that comprises a backbone that contains one or more rhamnogalacturonan-I stretches.

The term "alpha(1,4)-linked galacturonic acid stretch" refers to a stretch consisting of alpha(1→4)-galacturonic residues.

Besides RG-I domains, the RG-I polysaccharides of the present invention may contain one or more of the following domains:
- homogalacturonan (HG),
- xylogalacturonan (XG),
- apiogalacturonan (AG)
- rhamnogalacturonan-II (RG-II).

The domains XG, AG and RG-II typically represent only a minor fraction of the RG-I polysaccharides.

The HG domains, XG domains, AG and RG-II domains that are optionally present in the RG-I polysaccharides of the present invention comprise a backbone that consists of a linear chain of two or more α-(1-4)-linked D-galacturonic acids. The carboxyl groups of the galacturonic acid residues within the backbone of these domains may be esterified. Esterified galacturonic acid may occur in the form of the methyl ester or acetyl ester.

HG domains do not contain any sidechains.

The backbone of XG domains contains one or more sidechains in the form of D-xylose.

The backbone of AG domains contains one or more sidechains that are composed of one or more D-apiose residues.

The backbone of RG-II contains one or more side chains that are not exclusively composed of D-xylose or D-apiose.

The term "fruit" as used herein refers to the seed-beering structure in flowering plants.

The term "prebiotic" as used herein refers to substances that selectively induce the growth or activity of microorganisms that contribute to the well-being of their host.

The term "probiotic" as used herein refers to micro-organisms which, when orally administered in adequate amounts, provide a health benefit. These micro-organisms are selected from viable micro-organisms, non-viable micro-organisms, fragments of micro-organisms and combinations thereof.

The term "synbiotic" refers to a composition that contains a combination of (a) one or more prebiotics and (b) one or more probiotics.

The concentration of different polysaccharides and their monosaccharide composition can be determined by analytical techniques known to the skilled person. After acid hydrolysis, the monosaccharide composition can suitably be determined by High Performance Anion Exchange Chromatography combined with Pulse Amperometric Detection (HPAEC-PAD).

The molecular size distribution can be determined by High Performance Size-Exclusion Chromatography using refractive index (RI) detection (concentration), light scattering detection (molecular mass detection), UV detection (indicative for presence of proteins) and differential pressure detection (intrinsic viscosity detection).

The above mentioned analytical methods are described in: Analytical Biochemistry Vol. 207, Issue 1, 1992, pg 176 (for neutral sugar analysis) and in Mol. Nutr. Food Res., Vol 61, Issue 1, 2017, 1600243 (for the galacturonic acid analysis and the molecular size distribution).

All percentages mentioned herein, unless otherwise stated, refer to the percentage by weight.

The subject to which the RG-I polysaccharides containing composition of the present invention is orally administered preferably is a mammal, more preferably a human subject. According to a preferred embodiment, the human subject is an infant (< 4 year) still developing its core adult microbiota or an ageing person (>50 years) at risk of loosing the diversity and resilience of its core adult microbiota.

Oral administration within the context of the present method of treatment encompasses self-administration.

In accordance with a preferred embodiment, the subject that receives the orally administered RG-I polysaccharides containing composition is suffering from or at risk of suffering from a metabolic disorder. Most preferably, the subject is suffering from a metabolic disorder. Metabolic disorders that can successfully be treated (therapeutically or prophylactically) by the present treatment include overweight, obesitas, or insulin-resistance. The present treatment is particularly suitable for the therapeutic or prophylactic treatment of overweight or obesitas and insulin-resistance.

In accordance with another preferred embodiment, the subject is suffering from or at risk of suffering from intestinal barrier dysfunction. More preferably, the subject is suffering from intestinal barrier dysfunction.

The intestinal barrier, or intestinal mucosal barrier, refers to the property of the intestinal mucosa that ensures adequate containment of undesirable luminal contents within the intestine while preserving the ability to absorb nutrients. The separation it provides between the body and the luminal content of the gut prevents the uncontrolled translocation of luminal contents into the body. Its role in protecting the mucosal tissues and circulatory system from exposure to pro-inflammatory pathogens, toxins, and antigens is vital for the maintenance of health and well-being. Intestinal barrier dysfunction has been implicated in numerous health conditions such as: food allergies, microbial infections, irritable bowel syndrome, inflammatory bowel disease, celiac disease, metabolic syndrome, non-alcoholic fatty liver disease, diabetes, and septic shock.

In accordance with a preferred embodiment of the present invention, the prebiotic composition is used to therapeutically or prophylactically treat intestinal dysbiotic condition. The subject receiving treatment of an intestinal dysbiotic condition in accordance with the present invention preferably is a subject suffering from or at risk of suffering from a pathogenic intestinal dysbiotic condition, most preferably a subject suffering from such a pathogenic intestinal dysbiotic condition. Here "pathogenic" means that the condition is capable of causing or aggravating a disease.

In accordance with another preferred embodiment, the prebiotic composition is used to increase intestinal fermentative production of short chain fatty acids.

The RG-I polysaccharides that are employed in accordance with the present invention preferably originate from a plant source selected from apple, bell pepper, bilberry, carrot, citrus, grape, pea, chicory, sugar beet and olives, okra and combinations thereof. Even more preferably, the RG-I polysaccharides originate from a plant source selected from apple (e.g. apple pomace), bell pepper, carrot, citrus peel, grape, chicory, sugar beet (e.g.sugar beet pulp), olive (e.g. olive pulp), okra and combinations thereof. Most preferably, the RG-I polysaccharides originate from carrot or apple.

The RG-I polysaccharides are preferably incorporated in the prebiotic composition in the form of an pectic polysaccharide isolate that is enriched in RG-I polysaccharides. Accordingly, in a particularly preferred embodiment the RG-I polysaccharides represent at least 20 wt.%, more preferably at least 40 wt.%, even more preferably at least 50 wt.% and most preferably at least 60 wt.% of the pectic polysaccharides present in the prebiotic composition.

The RG-I polysaccharides have a backbone that comprises rhamnogalacturonan-I stretches and optionally alpha(1,4)-linked homo-galacturonic acid stretches. The molar ratio of galacturonic acid residues to rhamnose residues in the RG-I polysaccharides is within the range of 20:1 to 1:1. Preferably the molar ratio of galacturonic acid residues to rhamnose residues in the RG-I polysaccharides ranges from 15:1 to 1:1, more preferably from 12:1 to 1:1, even more preferably from 10:1 to 1:1, most preferably from 9:1 to 1:1.

Preferably, rhamnose residues represent 3-50%, more preferably 5-50% and most preferably 10-50% of the monosaccharide residues in the backbone of the RG-I polysaccharides.

Rhamnose residues typically represent 3-50%, more preferably 3.5-40% and most preferably 4-35% of all the monosaccharide residues contained in the RG-I polysaccharides. i.e. including the monosaccharide residues that are contained in sidechains.

Galacturonic acid residues typically represent 50-97%, more preferably 50-95% and most preferably 50-90% of the monosaccharide residues in the backbone of the RG-I polysaccharides.

Galacturonic acid residues typically represent 10-80%, more preferably 15-70% and most preferably 20-65% of all the monosaccharide residues contained in the RG-I polysaccharides. i.e. including the monosaccharide residues that are contained in sidechains.

The RG-I polysaccharides typically have a molecular weight of at least 20 kDa. Preferably, the RG-I polysaccharides havea molecular weight between 25 kDa and 2,000 kDa, more preferably between 30 kDa and 1,500 kDa, even more preferably between 35 kDa and 1,200 kDa, most preferably between 40 kDa and 1,000 kDa.

The average molecular weight of the RG-I polysaccharides that are contained in the present composition preferably exceeds 30 kDa, more preferably it exceeds 40 kDa and most preferably it exceeds 60 kDa.

Preferably, less than 85% of the galacturonic acid residues in the RG-I polysaccharides is esterified in the form of a methyl ester. More preferably, the RG-I polysaccharides have such a degree of esterification of between 0% and 70%, more preferably between 0% and 60%, even more preferably between 0% and 55%, most preferably between 0% and 50%.

Preferably, 0-95% of the galacturonic acid residues in the RG-I polysaccharides is esterified in the form of an acetyl ester. More preferably, the RG-I polysaccharides have such a degree of esterification of between 5% and 90%, more preferably between 7% and 50%, most preferably between 8% and 30%.

The backbone of the RG-I polysaccharides consists of galacturonic acid residues and rhamnose residues. If the RG-I polysaccharides comprises one or more side chains, the polysaccharide may additionally contain residues of arabinose and/or galactose. Furthermore, the sidechains of the RG-I polysaccharide may provide minor amounts of residues of the monomers fucose, glucose, glucuronic acid, xylose, and/or uronic acid. The one or more side chains preferably are selected from galactan side chains, arabinan side chains and arabinogalactan side chains.

The arabinan side chain comprises at least one or more alpha(1,5)-linked arabinose residues and is substituted at the 4-OH position of a rhamnose residues in the RG-I domain. The arabinan side chain may be linear or branched. In case the side chain is linear, the side chain consists of alpha(1,5)-linked arabinose residues. In case the arabinan side chain is a branched side chain, one or more alpha-arabinose residues are linked to the 2-OH and/or 3-OH of alpha(1,5)-linked arabinoses. The length of the arabinan side chain (expressed as number of monomer units) preferably is between 1 and 100 monomer units, more preferably between 1 and 50 units, even more preferably between 1 and 30 units.

The galactan side chain comprises at least one or more beta(1,4)-linked galactose residues and is substituted at the 4-OH position of a rhamnose residues in the RG-I domain.

The galactan side chain preferably is substantially linear (unbranched), i.e. less than 10 mol% of galactose residues within the chain are beta(1,3)-linked or beta(1,6)-linked galactose residues, preferably less than 5 mole%, preferably less than 2 mole%, preferably less than 1 mole%. The length of the galactan side chain preferably is between 1 and 100 monomer units, more preferably between 1 and 50 units, even more preferably between 1 and 30 units.

The arabinogalactan side chain is substituted at the 4-OH position of a rhamnose residue in the RG-I domain and can be a type I arabinogalactan (AGI) or a type II arabinogalactan AGII). AGI is composed of a (1→4)-β-D-Gal*p* backbone on which substitutions by monomeric Gal*p* units at the *O-6* or at the *O-3* position can occur. AGI is further substituted with α-L-Ara*f-p* residues and/or with (1→5)-α-L-Ara*f* short side chains. AGII is composed of α(1→3)-β-D-Gal*p* backbone decorated with (1→6)-β-D-Gal*p* secondary chains, which are arabinosylated.

Preferably the molar ratio of arabinose residues to rhamnose residues in the RG-I polysaccharide does not exceed 30:1, more preferably it does not exceed 15:1, even more preferably it does not exceed 8:1 and most preferably it does not exceed 5:1.

The molar ratio of galactose residues to rhamnose residues in the RG-I polysaccharide preferably does not exceed 30:1, more preferably it does not exceed 15:1, even more preferably it does not exceed 8:1 and most preferably it does not exceed 5:1.

According to a preferred embodiment, at least 20% of the rhamnose residues in the RG-I stretches is substituted at the 4-OH position. More preferably at least 30%, even more preferably at least 40%, most preferably at least 45% of these rhamnose residues is substituted at the 4-OH position. Preferably at most 90%, more preferably at most 80% of these rhamnose residues is substituted at the 4-OH position.

The prebiotic composition for use in the present method preferably contains at least 0.2%, more preferably 0.3-10% and most preferably 0.4-5% by weight of dry matter of the RG-I polysaccharides as defined herein.

The prebiotic composition for use in the present method advantageously contains one or more other prebiotics besides the RG-I polysaccharides. Preferably, the composition contains at least 1% by weight of dry matter, more preferably at least 3% by weight of dry matter of one or more prebiotics selected from lactulose, inuline, fructooligosaccharides, galactooligosaccharides, milk oligosaccharides, guar gum and gum Arabic.

The RG-I polysaccharides are believed to be particularly effective in the present method of treatment if it is no longer entangled in the matrix of cell wall material. Accordingly, in a particularly preferred embodiment, the RG-I polysaccharides containing composition of the present invention contains at least 0.05% by weight of dry matter, more preferably at least 0.1% by weight of dry matter, even more preferably at least 0.2% by weight of dry matter and most preferably at least 0.3% by weight of dry matter of readily water-soluble RG-I polysaccharides. The concentration of readily water-soluble RG-I polysaccharides in the RG-I polysaccharides containing composition can be determined by combining 100 ml of demineralized water (20°C) with a sufficient amount of the RG-I polysaccharides containing composition to provide 2.5 grams of dry matter, followed by stirring for 5 minutes and filtration over a 100 µm filter. The RG-polysaccharides in the filtrate are readily water-soluble RG-I polysaccharides.

In accordance with a particularly preferred embodiment of the present invention the present composition is orally administered to the subject during a period of at least 2 days in an amount providing at least 1 mg RG-I polysaccharides per kg of bodyweight per day. More preferably, the amount of at least 4 mg RG-I polysaccharides per kg of bodyweight per day, even more preferred at least 15 mg/kg of bodyweight per day, most preferred at least 20-100 mg/kg RG-I polysaccharides per kg of bodyweight per day is provided during a period of at least 7 days, most preferably during a period of at least 14 days, by feeding the composition containing the RG-I polysaccharides.

In accordance with another preferred embodiment, the RG-I polysaccharides containing composition is orally administered to the subject during a period of at least 21 days, to provide the RG-I polysaccharides in an amount of at least 4 mg RG-I polysaccharides per kg of bodyweight per day, more preferably 15-300 mg RG-I polysaccharides per kg of bodyweight per day.

The prebiotic composition of the present invention was found to be capable of inducing the growth of intestinal micro-organisms that are believed to provide health benefits, notably of *Akkermansia muciniphila* and *Bifidobacterium spp.* Accordingly, in another preferred embodiment, the RG-I polysaccharides containing composition is capable of inducing growth or activity of *Akkermansia muciniphila* and/or *Bifidobacterium* spp. within the subject's intestinal microbiota. Most preferably, the composition is capable of inducing growth or activity of *Akkermansia muciniphila* within the subject's intestinal microbiota.

The prebiotic composition of the present invention was found to be capable of inducing the production of short chain fatty acids by the intestinal microbiota, while this is accompanied with substantially less production of gas compared to classical prebiotics such as inulin. Accordingly, in another preferred embodiment, the RG-I polysaccharides containing composition is capable of enhancing the intestinal fermentative production of short chain fatty acids with reduced side effects, such as intestinal discomfort, flatulence and regurgitation, associated with rapid gas production.

According to a particularly preferred embodiment the composition is selected from a drink, a oral dosage unit, a powder, a bar and a spread.

The drink typically is a liquid. Preferably the drink contains at 80 wt.%, more preferably at least 85 wt.% water. The drink preferably contains at least 1.5 g/l, more preferably at least 3 g/l and most preferably 5-200 g/l of the RG-I polysaccharides.

The oral dosage unit preferably is a capsule or a tablet. The oral dosage unit preferably has weight in the range of 50 to 1500 milligrams, more preferably of 100 to 800 milligrams. The oral dosage unit typically contains at least 1 wt.%, more preferably at least 20 wt.% and most preferably 40-90 wt.% of the RG-I polysaccharides.

The composition in the form of a powder preferably is a water-soluble powder that can be used to prepare a beverage. Typically, the powder contains at least 0.5 wt.%, more preferably at least 5 wt.% and most preferably 10-75 wt.% of the RG-I polysaccharides.

The composition in the form of a bar preferably is a bar, preferably a bar having a weight in the range of 10 to 200 grams, more preferably of 25 to 100 grams. The bar typically contains at least 0.1 wt.%, more preferably at least 0.5 wt.% and most preferably 1- 20 wt.% of the RG-I polysaccharides.

The composition in the form of a spread preferably is a water-in-oil emulsion, preferably a water-in-oil emulsion comprising 20-90 wt.% of a fat phase and 10-80 wt.% of an aqueous phase. The spread preferably contains at least 0.3 wt.%, more preferably at least 1-10 wt.% and most preferably 1.5-16 wt.% of the RG-I polysaccharides.

Also disclosed is a prebiotic composition comprising:
- at least 0.1% by weight of dry matter of the RG-I-polysaccharides as defined herein before; and
- at least 1%, preferably at least 3% by weight of dry matter of one or more prebiotics selected from lactulose, inuline, fructooligosaccharides, galactooligosaccharides, milk oligosaccharides, guar gum and gum Arabic.

Even more preferaly, the product contains at least 1% by weight of dry matter, more preferably at least 3% by weight of dry matter of one or more prebiotics selected from lactulose, inuline, fructooligosaccharides, galactooligosaccharides and milk oligosaccharides. Preferred embodiments of the prebiotic composition are the same as described herein before in relation to the prebiotic composition for use in the present method of treatment.

Yet another aspect of the invention relates to a synbiotic composition comprising:
- at least 0.1% by weight of dry matter of the RG-I-polysaccharides as defined herein before; and
- 10⁴ to 10¹⁰ cfu/gram of *Akkermansia muciniphila* and/or 10⁶ to 10¹⁰ cfu/gram of *Bifidobacterium spp.*

Preferred embodiments of the synbiotic composition are the same as described herein before in relation to the prebiotic composition for use in the present method of treatment. The one or more probiotic microbial strains in the symbiotic composition preferably are live microbial strains, more preferably live bacterial strains.

The synbiotic composition preferably contains the one or more probiotic microbial strains in a concentration of 10⁴ to 10¹⁰ cfu, or the equivalent thereof in case the probiotic microbial strains are applied in non-viable form. More preferably, the symbiotic composition contains the one or more probiotic microbial strains in a concentration of 10⁵ to 10⁹ cfu or the equivalent thereof.

*Akkermansia muciniphila* is preferably contained in the probiotic composition in a concentration of 10⁵ to 10¹⁰ cfu/gram, more preferably 10⁶ to 10⁹ cfu/gram.

*Bifidobacterium spp.* is preferably contained in the probiotic composition in a concentration of 10⁶ to 10¹⁰ cfu/gram, more preferably 10⁷ to 10⁹ cfu/gram .

The invention is further illustrated by the following non-limiting examples.

### EXAMPLES

### Example 1

A RG-I polysaccharide fraction was isolated from dry bell pepper powder (Paprika Mild 80-100 Atsa Steamtr- Felix Reverte S.A.) at pilot plant scale using the procedure described below.

The bell pepper material (100 kg) was washed three times under gentle steering with 80% aqueous ethanol, i.e. twice at 80°C for 2 hours and then overnight at room temperature; each time using 12.5% (w/v), to remove ethanol soluble material. The ethanol insoluble residue was recovered every time by centrifugation (1000 G for 10 min). The ethanol insoluble residue obtained after the 3 wash cycles was dried and 90 kg was extracted twice with 1000 L hot water having a temperature of 95°C for 90 minutes. Each time, the supernatant was retained after centrifugation at 1000 G for 10 minutes. The collected supernatant was subsequently filtrated through cloth, and ultrafiltrated using 2 KDa molecular weight cut off membranes to remove small molecular weight material. A dry RG-I enriched extract was obtained by freeze drying the retentate, yielding approximately 5 kg of dry RG-I enriched polysaccharide extract.

### Characterisation of RG-I polysaccharide enriched extract

### Molecular mass distribution:

The molecular mass distribution of the polysaccharide samples was determined by High Performance Size-Exclusion Chromatography using refractive index (RI) detection (concentration), light scattering detection (molecular mass detection), UV detection (indicative for presence of proteins) and differential pressure detection (intrinsic viscosity detection). Pullulan molecular-mass standards were used for calibration.

### Monosaccharide composition:

Polysaccharide samples were dissolved in 0.5 M aqueous trifluoroacetic acid and hydrolyzed at 120°C for 2 hours. Samples were then neutralized with NaOH, and kept frozen until analyzed by high-pH anion- exchange chromatography (HPAEC) using pulsed-amperometric detection (PAD). Uronic acid in the samples was determined by using the colorimetric m-hydroxydiphenyl assay (Achmed & Labavitch, J. Food Biochem, 1978, 361)) automated on an autoanalyser (Skalar)

### Degree of esterification

Polysaccharide samples were treated with sodium hydroxide (0.25 M, 5 hr, 20°C) and then neutralized. Released methanol was measured as absorbance at 420 nm, after incubation with alcohol oxidase combined with developing reagent (acetyl acetone and acetic acid in 2M ammonium acetate). Released acetic acid was determined using the K-ACETAF acetic acid assay kit (Megazyme). Sugar beet pectin with known degree of methylation and acetylation was used as standard. Degree of esterification is expressed as molar amount of methanol and acetic acid released as percentage of the amount of uronic acid.

The molecular characteristics of the RG-I polysaccharide fraction is shown in Tables 1a and 1b.

**Table 1a**

| **Monosaccharides (%mol/mol)** | **Red Bell Pepper** |
|---|---|
| Rha (Rhamnose) | 5.0 |
| GalA (Galacturonic acid) | 70.0 |
| Ara (Arabinose) | 9.0 |
| Gal (Galactose) | 9.0 |
| Glc (Glucose) | 3.0 |
| Xyl (Xylose) | 2.0 |

**Table 1b**

| **Molecular Ratios** | **Red Bell Pepper** |
|---|---|
| GalA/Rha | 14 |
| Ara/Rha | 1.8 |
| Gal/Rha | 1.8 |
| Degree of Methylation % | 39 |
| Degree of Acetylation % | 9.0 |

### Example 2

Six week old specific pathogen free female C57BL/6 mice received *ad libitum* sterilized drinking water and a semi-synthetic irradiated AIN-93G diet (Research Diet Services, Wijk bij Duurstede, The Netherlands) with 30 mmol/kg calcium (uninfected and infected group) or the same food supplemented with 1% (w/w) of the RG-I enriched extract from bell pepper.

Mice were randomly assigned to treatment groups and housed with 3 mice per cage for 2 weeks and then individually for another week. The composition of the AIN-93G diet is described by Reeves et al. (AIN-93 purified diets for laboratory rodents: final report of the American Institute of Nutrition ad hoc writing committee on the reformulation of the AIN-76A rodent diet. J Nutr (1993)123: 1939-1951).

Faeces was collected before the dietary intervention at baseline and after 3 weeks of dietary intervention. Genomic DNA was extracted from fecal samples following the manufacturer's protocol (Zymo research). Isolated gDNA was subjected to microbial identification by 16S rRNA gene sequencing. Specific primers were used to PCR amplify the genomic region of interest, like the V3-V4 or the V4 hyper variable regions of the 16S rRNA gene. Paired-end sequence reads were generated using the Illumina MiSeq system. FASTQ sequence files were generated using the Illumina Casava pipeline version 1.8.3. Initial quality assessment was based on the Illumina Chastity filtering. Subsequently, reads containing PhiX control signal were removed using an in-house filtering protocol (Baseclear). In addition, reads containing (partial) adapters were clipped (up to minimum read length of 50bp). The second quality assessment was based on the remaining reads using the FASTQC quality control tool version 0.10.0.

### Bacterial DNA sequencing

The Illumina Miseq data were analyzed with a workflow employing the Quantitative Insights Into Microbial Ecology (QIIME, v8) pipeline (Caporaso et al., QIIME allows analysis of high-throughput community sequencing data, Nature Methods (2010), 7(5), 335-336 and Edgar, Search and clustering orders of magnitude faster than BLAST, Bioinformatics (Oxford, England), (2010), 26(19), 2460-2461. The data was demultiplexed and filtered for not matching barcodes and small fragments (>50nt). Open reference OTU picking was performed in QIIME using the Silva 111 database and chimeras were detected by USEARCH and filtered out of the OTUs. From the filtered OTUs a biom file and phylogenetic tree file was generated using again the Silva 111 database. Further outputs were generated via QIIME, such as filtered reads per sample, PD whole tree diversity measurements and the level 1 to 6 taxonomic distributions with relative abundances.

### Impact of RG-I enriched polysaccharide extract on microbial composition

Individual faecal samples of 12 mice per group were subjected to Illumina 16S rRNA sequencing to get further insights in the microbial composition. Between 26x10³ and 79x10⁴ reads were obtained per sample. The results are shown in Tables 2, 3 and 4.

**Table 2**

| | Abundance % | | *Ratio Firmicutes : Bacteriodetes* |
|---|---|---|---|
| | *Firmicutes* | *Bacteriodetes* | |
| Control baseline | 0.459 ± 0.184 | 0.436 ± 0.144 | 1.336 ± 1.059 |
| RG-I baseline | 0.554 ± 0.117 | 0.381 ± 0.089 | 1.596 ± 0.684 |
| control 3 wks | 0.489 ± 0.225 | 0.361 ± 0.235 | 1.350 ± 1.459 |
| RG-I 3 wks | 0.502 ± 0.190 | 0.178 ± 0.124* | 4.239 ± 2.648* |

| | | | |
|---|---|---|---|
| * denotes significance vs RG-I baseline | | | |

**Table 3**

| | Abundance % | |
|---|---|---|
| | *Actinobacteria* | *Verrucomicrobia* |
| Control baseline | 0.083 ± 0.080 | 0.000 ± 0.000 |
| RG-I baseline | 0.036 ± 0.044 | 0.002 ± 0.006 |
| control 3 wks | 0.071 ± 0.084 | 0.002 ± 0.006 |
| RG-I 3 wks | 0.231 ± 0.159* | 0.036 ± 0.022* |

| | | |
|---|---|---|
| * denotes significance vs RG-I baseline | | |

**Table 4**

| | Abundance % | |
|---|---|---|
| | *Bifidobacterium* | *Akkermansia* |
| Control baseline | 0.003 ± 0.001 | 0.000 ± 0.000 |
| RG-I baseline | 0.001 ± 0.001 | 0.002 ± 0.006 |
| control 3 wks | 0.028 ± 0.060 | 0.002 ± 0.006 |
| RG-I 3 wks | 0.134 ± 0.110* | 0.041 ± 0.027* |

| | | |
|---|---|---|
| * denotes significance vs RG-I baseline | | |

On phyla level the addition of RG-I enriched polysaccharide extract in the diet significantly increased abundances of *Actinobacteria* and *Verrucomicrobia,* while *Bacteroidetes* and *Proteobacteria* decreased in their abundances (> 0.5 % abundances; Wilcoxon, P<0.05) resulting in an increased ratio of *Firmicutes* / *Bacteroidetes.*

On genus level the redundancy analysis indicated that the addition of RG-I enriched polysaccharide extract in the diet had a significant impact on the microbial composition (P< 0.05; Monte Carlo permutation). The inclusion of RG-I enriched polysaccharide extract in the diet lead to higher abundances of the *Bifidobacterium (Phylum Actinobacteria), Allobaculum (Phylum Firmicutes), Akkermansia (Phylum Verrucomicrobia)* and the Unassigned bacteria group.

### Example 3

Non-digestible polysaccharides are mainly digested in the human large intestine by the gut microbiota. This may lead to the growth of health beneficial bacteria, lower the pH, increase resistance to intestinal pathogens and modulate the metabolic activity of the microbiota. Beneficial (prebiotic) functional carbohydrates will promote the production of health beneficial metabolites such as short chain fatty acids (SCFA, i.e. acetate, propionate and butyrate) while reducing the production of unwanted metabolites of protein metabolism such as branched SCFA and ammonia.

Plant derived polysaccharide extracts were tested for their impact on the metabolic activity of the microbiota using an established short-term colonic incubation model.

At the start of the incubation, a sugar-depleted base colon medium containing nutrients that are present in the colon (e.g. host-derived glycans such as mucin) was introduced into 70 mL penicillin bottles, already containing the test extracts (5 g/L final concentration). The bottles were sealed with rubber stoppers and anaerobiosis was obtained by flushing with N₂. Subsequently, a human fecal inoculum was prepared by mixing a freshly collected fecal sample with anaerobic phosphate buffer. After homogenization and removal of particles via centrifugation (2 min, 500 g), the faecal inoculum was added to the different bottles. At that point, the incubation started for a period of 48h during which temperature was controlled at 37 °C and continuous mixing was ensured by a shaker (90 rpm). Samples were taken after 6, 24 and 48 h incubation for pH (Senseline F410; ProSense, Oosterhout, The Netherlands), gas pressure (Hand-held pressure indicator CPH6200; Wika, Echt, The Netherlands) and SCFA analysis. SCFA, being acetate, propionate, butyrate and branched SCFA (isobutyrate, isovalerate and isocaproate), were measured as described by De Weirdt et al (Human faecal microbiota display variable patterns of glycerol metabolism, FEMS Microbiol. Ecol. 2010, 74, 601-611.).

The sugar-depleted colon medium and Inulin, a well known prebiotic (Beneo, DP ≥23, ~100% inulin), were used as negative and positive reference, respectively.

Sample A was produced from bell pepper powder (Paprika poeder, Natural Spices Mijdrecht, the Netherlands) by aqueous extraction (10% w/w, 2hr 90 °C), centrifugation to remove non-soluble residues, filtration (40 kDa cut off) to remove small molecules and drying to obtain a powder.

Sample B was produced from dried carrot pomace, the residue of carrot juice production (carrot fibre powder, GreendFields, Poland). Sample B was produced by aqueous extraction (10% w/w 2 hr 45 °C) using pectinase (Pectinex^{®} Ultra Mash, Novozymes), heat inactivation (90 °C, 10 min), removal of non-soluble residues by decanting, ultrafiltration (40kDa cut of) and finally drying.

Sample C was extracted from apple pomace powder (apple pomace, GreendFields, Poland) in the same manner as sample B.

Sample D was produced from Okra powder (Ground Okra, My Foods, Blue mountain peak, UK) using hot water extraction (10% w/w, 2hr at 90°C), dialysis against water for several hours to remove the small molecular weight material. The dialyzed extract was then freeze dried. Determination of monosaccharide composition of the aforementioned samples was performed as described for example 1. The results are shown in Table 5.

**Table 5**

| **Source** | | **A Bell pepper** | **B Carrot** | **C Apple** | **D Okra** |
|---|---|---|---|---|---|
| Rha (Rhamnose) | %mol/mol | 8 | 17 | 8 | 31 |
| GalA (Galacturonic acid) | %mol/mol | 50 | 23 | 18 | 40 |
| Ara (Arabinose) | %mol/mol | 13 | 23 | 48 | 1 |
| Gal (Galactose) | %mol/mol | 14 | 33 | 9 | 22 |
| Glc (Glucose) | %mol/mol | 9 | 2 | 10 | 6 |
| Xyl (Xylose) | %mol/mol | 3 | 2 | 6 | 0 |
| *Total* | | *98* | *100* | *99* | *100* |
| GalA/Rha | | 6.0 | 1.4 | 2.2 | 1.3 |
| Ara/Rha | | 1.5 | 1.4 | 5.7 | 0.0 |
| Gal/Rha | | 1.7 | 1.9 | 1.1 | 0.7 |
| % Methylation | | 39 | 16 | 27 | n.d. |

The results of the incubation experiments are shown in Table 6.

**Table 6**

| | | Medium | Inulin | **A** | **B** | **C** | **D** |
|---|---|---|---|---|---|---|---|
| | | | | Bell pepper | Carrot | Apple | Okra |
| pH decrease | | 0.01 | 0.66 | 0.39 | 0.63 | 0.64 | 0.52 |
| SCFA production | mM | 27.8 | 51.1 | 49.3 | 56.4 | 55.6 | 52.9 |
| Gas production | kPa | 28.6 | 86.5 | 47.9 | 49.5 | 57.2 | 57.1 |
| Branched SCFA | mM | 2.8 | 1.2 | 1.9 | 1.3 | 1.1 | 1.7 |
| Ammonium | mg/L | 373 | 169 | 346 | 221 | 214 | 287 |

Results show that all four plant derived RG-I polysaccharide extracts were fermented readily by the gut microbiota.

All RG-I polysaccharide extracts increased production of SCFA. As a matter of fact all extracts were found ot increase SCFA production to a level similar to or in excess of the level observed for inulin.

All RG-I polysaccharide extracts decreased the production of branched SCFA and ammonia. Surpisingly, gas production observed for all RG-I polysaccharide extracts was substantially lower than the gas production observed for inulin. Excessive gas production may lead to gut discomfort and bloating, a well described unwanted side effect of most classical prebiotics, including inulin.

### Example 4

A pasteurized dairy drink is prepared on the basis of the recipe shown in Table 7.

**Table 7**

| | **Wt.%** |
|---|---|
| Milk fat | 1 |
| Alkalized cocoa | 0.1 |
| Sucrose | 3 |
| RG-I polysaccharide isolate ¹ | 2 |
| Inulin | 2 |
| Carrageenan | 0.01 |
| High acyl gellan | 0.05 |
| Hydroxypropyl starch | 0.2 |
| Carboxymethyl cellulose | 0.1 |
| Skim milk | Remainder |

| | |
|---|---|
| ¹ isolated from dried carrot pomace (see Example 3) | |

Consumption of 200 ml of this dairy drink per day by a human adult improves intestinal health and provides extra protection against common cold and flu.

### Example 5

A nutritional bar (45 g) is prepared on the basis of the recipe shown in Table 8.

**Table 8**

| | **Wt.%** |
|---|---|
| Maltodextrin | 12.9 |
| Milk protein isolate | 9.0 |
| Soy protein isolate | 0.5 |
| Rice flour | 5.3 |
| Oat bran | 14.4 |
| Rice Crisps | 9.0 |
| Crystalline fructose | 6.8 |
| Evaporated cane juice syrup | 27.7 |
| Salt | 0.3 |
| Glycerine | 1.0 |
| Almond butter | 3.3 |
| RG-I polysaccharide isolate ¹ | 4.5 |
| Inulin | 1 |
| Vitamins/mineral mix | 3.1 |
| Vanilla | 1.2 |

| | |
|---|---|
| ¹ isolated from dried carrot pomace (see Example 3) | |

The bar is produced as follows: All wet ingredients are mixed together (syrup, glycerine, almond butter and flavours) at 50° C.

Separately, dry ingredients are mixed together, then the wet slurry is added to the dry mix and the mass is mixed for 2 to 5 minutes under high shear. The dough is slabed and cut into bar shape before packing .

Consumption of 2 bars per day by a human adult improves intestinal health and provides extra protection against common cold and flu.

### Example 6

A dietary supplement is prepared in the form of a capsule containing the dry mix shown in Table 9.

**Table 9**

| | **mg** |
|---|---|
| RG-I polysaccharide isolate ¹ | 300 |
| D-alpha-tocopherol | 10 |
| Oligomeric proanthocyanidin | 15 |
| Vitamin B1 | 1.4 |
| Vitamin B2 | 1.6 |
| Niacin (vitamin B3) | 18.0 |
| Pantothenic acid (vitamin B5) | 6.0 |
| Vitamin B6 | 2.0 |
| Vitamin B12 | 1.0 |
| Folic acid (vitamin B9) | 0.2 |
| Biotin | 0.15 |
| Microcrystalline cellulose | 30 |

| | |
|---|---|
| ¹ isolated from dried carrot pomace (see Example 3) | |

### Example 7

RG-I polysaccharide fractions were extracted from different source materials for testing in the short-term colonic incubation model described in Example 3. The sugar-depleted colon medium and inulin, a well known prebiotic (Beneo, DP ≥23, ~100% inulin), were used as negative and positive reference, respectively.

Sample A was produced from dried and milled pea hulls (ex Cosucra, Warcoing, Belgium). The powder was dispersed in demineralised water (100 g/L) and subjected to enzymatic pre-hydrolysis with a thermostable alpha-amylase (Megazyme) at 90°C for 30 min and further hydrolysis using pectinase (2 hr 45 °C, 0,2 v/v% Pectinex^{®} Ultra Mash, Novozymes). Enzymolysis was terminated by heating at 100°C for 10 min, followed by centrifugation (18.000 g, 10 min) and extensive dialysis of the supernatant using a membrane with a 12-14 kDa (Visking, London, UK) cut off. The material was then lyophilized.

Sample B was produced using the same method from dried and milled sugar beet pulp ( ex Suiker Unie, Dinteloord, NL), omitting however the α-amylase pre-incubation step.

Sample C was produced using the same method from dried and milled chicory pulp (ex Cosucra, Warcoing, Belgium), omitting however the α-amylase pre-incubation step.

The monosaccharide composition of the aforementioned samples was determined using the method described in Example 1. The results are shown in Table 10.

**Table 10**

| | | **A** | **B** | **C** |
|---|---|---|---|---|
| Source | | **Pea** | **Sugar beet** | **Chicory** |
| Rha (Rhamnose) | %mol/mol | 8 | 7 | 8 |
| GalA (Galacturonic acid) | %mol/mol | 57 | 40 | 35 |
| Ara (Arabinose) | %mol/mol | 14 | 45 | 48 |
| Gal (Galactose) | %mol/mol | 6 | 7 | 8 |
| Glc (Glucose) | %mol/mol | 8 | 1 | 1 |
| Xyl (Xylose) | %mol/mol | 6 | 0 | 0 |
| *Total* | | | | |
| GalA/Rha | | 7.1 | 5.7 | 4.4 |
| Ara/Rha | | 1.8 | 6.4 | 6.0 |
| Gal/Rha | | 0.8 | 1.0 | 1.0 |
| % Methylation | | 33 | 26 | 27 |

The results of the incubation experiments are shown in Table 11.

**Table 11**

| | | | | **A** | **B** | **C** |
|---|---|---|---|---|---|---|
| | | Medium | Inulin | **Pea** | **Sugar beet** | **Chicory** |
| pH decrease | | 0.00 | 0.61 | 0.47 | 0.62 | 0.66 |
| SCFA production | mM | 27.5 | 59.7 | 61.3 | 66.4 | 61.7 |
| Gas production | kPa | 31.9 | 87.6 | 62.6 | 70.0 | 74.6 |
| Branched SCFA | mM | 3.4 | 0.5 | 3.0 | 3.0 | 1.9 |
| Ammonium | mg/L | 362 | 181 | 316 | 293 | 228 |

These results show that these different plant derived RG-I polysaccharide extracts were readily fermented by the gut microbiota.

All RG-I polysaccharide extracts increased production of SCFA. As a matter of fact all extracts were found ot increase SCFA production to a level similar to or in excess of the level observed for inulin.

All RG-I polysaccharide extracts decreased the production of branched SCFA and ammonia compared to the medium control.

## Claims

1. A prebiotic composition for use in a method of therapeutic or prophylactic treatment of disorders associated with disturbed composition or functionality of the intestinal microbiome in a subject, the disorder being selected from overweight, obesitas, insulin-resistance and intestinal barrier dysfunction, said use comprising oral administration of the prebiotic composition to the subject, wherein the composition contains at least 0.1% by weight of dry matter of rhamnogalacturonan I (RG-I) polysaccharides originating from fruit, carrot, pea, chicory or sugar beet, said RG-I polysaccharides having a molecular weight in excess of 15 kDa and having a backbone consisting of galacturonic acid residues and rhamnose residues, said rhamnose residues being contained in alpha(1-4)-galacturonic-alpha(1-2)-rhamnose residues, wherein the molar ratio of galacturonic acid residues to rhamnose residues in the RG-I polysaccharides is within the range of 20:1 to 1:1.

2. Prebiotic composition for use according to claim 1, wherein the composition is ingested by the subject during a period of at least 3 days in an amount providing at least 1 mg RG-I polysaccharides per kg of bodyweight per day.

3. Prebiotic composition for use according to claim 1 or 2, wherein the RG-I polysaccharides represent at least 20 wt.% of the pectic polysaccharides present in the prebiotic composition.

4. Prebiotic composition for use according to any one of the preceding claims, wherein the molar ratio of galacturonic acid residues to rhamnose residues in the RG-I polysaccharides does not exceed 15:1, preferably does not exceed 12:1, more preferably does not exceed 10:1.

5. Prebiotic composition for use according to any one of the preceding claims, wherein the molar ratio of arabinose residues to rhamnose residues in the RG-I polysaccharide does not exceed 30:1.

6. Prebiotic composition for use according to any one of the preceding claims, wherein the molar ratio of galactose residues to rhamnose residues in the RG-I polysaccharide preferably does not exceed 30:1.

7. Prebiotic composition for use according to any one of the preceding claims, wherein less than 85% of the galacturonic acid residues in the RG-I polysaccharides is esterified in the form of methyl esters.

8. Prebiotic composition for use according to any one of the preceding claims, wherein the RG-I polysaccharides originate from a plant source selected from carrot, apple, bell pepper, citrus, bilberry, grape, pea, chicory, sugar beet, olive, okra and combinations thereof.

9. Prebiotic composition for use according to any one of the preceding claims, wherein the subject is suffering from or at risk of suffering from a metabolic disorder.

10. Prebiotic composition for use according to any one of claims 1-8, wherein the subject is suffering from or at risk of suffering from intestinal barrier dysfunction.

11. A synbiotic composition comprising:
• at least 0.1% by weight of dry matter of rhamnogalacturonan I (RG-I) polysaccharides originating from carrot or apple, said RG-I polysaccharides having a molecular weight in excess of 15 kDa and having a backbone consisting of galacturonic acid residues and rhamnose residues, said rhamnose residues being contained in alpha(1-4)-galacturonic-alpha(1-2)-rhamnose residues, wherein the molar ratio of galacturonic acid residues to rhamnose residues in the RG-I polysaccharides is within the range of 20:1 to 1:1; and
• 10⁵ to 10¹⁰ cfu/gram of *Akkermansia muciniphila* and/or 10⁶ to 10¹⁰ cfu/gram of *Bifidobacterium spp.*

12. A composition according to claim 11 or a composition for use according to any one of claims 1-10, wherein the composition is selected from a drink, a capsule, a tablet, a powder, a bar and a spread.

## Patentansprüche

1. Präbiotische Zusammensetzung zur Verwendung in einem Verfahren zur therapeutischen oder prophylaktischen Behandlung von Erkrankungen im Zusammenhang mit einer gestörten Zusammensetzung oder Funktionalität des intestinalen Mikrobioms bei einem Subjekt, wobei die Erkrankung ausgewählt ist aus Übergewicht, Fettleibigkeit, Insulinresistenz und Dysfunktion der Darmbarriere, wobei die Verwendung die orale Verabreichung der präbiotischen Zusammensetzung an das Subjekt umfasst, wobei die Zusammensetzung mindestens 0,1 Gew.-% Trockenmasse an Rhamnogalacturonan-I(RG-I)-Polysacchariden aus Frucht, Karotte, Erbse, Zichorie oder Zuckerrübe enthält, wobei die RG-I-Polysaccharide ein Molekulargewicht von mehr als 15 kDa aufweisen und eine Hauptkette aufweisen, die aus Galacturonsäureresten und Rhamnoseresten besteht, wobei die Rhamnosereste in alpha(1→4)-Galacturonsäure-alpha(1→2)-Rhamnoseresten enthalten sind, wobei das Molverhältnis von Galacturonsäureresten zu Rhamnoseresten in den RG-I-Polysacchariden im Bereich von 20:1 bis 1:1 liegt.

2. Präbiotische Zusammensetzung zur Verwendung nach Anspruch 1, wobei die Zusammensetzung durch das Subjekt während eines Zeitraums von mindestens 3 Tagen in einer Menge eingenommen wird, die mindestens 1 mg RG-I-Polysaccharide pro kg Körpergewicht pro Tag bereitstellt.

3. Präbiotische Zusammensetzung zur Verwendung nach Anspruch 1 oder 2, wobei die RG-I-Polysaccharide mindestens 20 Gew.-% der pektischen Polysaccharide darstellen, die in der präbiotischen Zusammensetzung vorhanden sind.

4. Präbiotische Zusammensetzung zur Verwendung nach einem der vorhergehenden Ansprüche, wobei das Molverhältnis von Galacturonsäureresten zu Rhamnoseresten in den RG-I-Polysacchariden 15:1 nicht übersteigt, vorzugsweise 12:1 nicht übersteigt, besonders bevorzugt 10:1 nicht übersteigt.

5. Präbiotische Zusammensetzung zur Verwendung nach einem der vorhergehenden Ansprüche, wobei das Molverhältnis von Arabinoseresten zu Rhamnoseresten in dem RG-I-Polysaccharid 30:1 nicht übersteigt.

6. Präbiotische Zusammensetzung zur Verwendung nach einem der vorhergehenden Ansprüche, wobei das Molverhältnis von Galactoseresten zu Rhamnoseresten in dem RG-I-Polysaccharid vorzugsweise 30:1 nicht übersteigt.

7. Präbiotische Zusammensetzung zur Verwendung nach einem der vorhergehenden Ansprüche, wobei weniger als 85 % der Galacturonsäurereste in den RG-I-Polysacchariden in der Form von Methylestern verestert sind.

8. Präbiotische Zusammensetzung zur Verwendung nach einem der vorhergehenden Ansprüche, wobei die RG-I-Polysaccharide aus einer Pflanzenquelle stammen, die aus Karotte, Apfel, Paprikaschote, Zitrusfrucht, Heidelbeere, Traube, Erbsen Zichorie, Zuckerrübe, Olive, Okra und Kombinationen davon ausgewählt ist.

9. Präbiotische Zusammensetzung zur Verwendung nach einem der vorhergehenden Ansprüche, wobei das Subjekt an einer Stoffwechselerkrankung leidet oder gefährdet ist, daran zu leiden.

10. Präbiotische Zusammensetzung zur Verwendung nach einem der Ansprüche 1-8, wobei das Subjekt an einer Dysfunktion der Darmbarriere leidet oder gefährdet ist, daran zu leiden.

11. Synbiotische Zusammensetzung, umfassend:
• mindestens 0,1 Gew.-% Trockenmasse an Rhamnogalacturonan-I(RG-I)-Polysacchariden aus Karotte oder Apfel, wobei die RG-I-Polysaccharide ein Molekulargewicht von mehr als 15 kDa aufweisen und eine Hauptkette aufweisen, die aus Galacturonsäureresten und Rhamnoseresten besteht, wobei die Rhamnosereste in alpha(1→4)-Galacturonsäure-alpha(1→2)-Rhamnoseresten enthalten sind, wobei das Molverhältnis von Galacturonsäureresten zu Rhamnoseresten in den RG-I-Polysacchariden im Bereich von 20:1 bis 1:1 liegt; und
• 10⁵ bis 10¹⁰ KBE/Gramm *Akkermansia muciniphila* und/oder 10⁶ bis 10¹⁰ KBE/Gramm *Bifidobacterium spp.*

12. Zusammensetzung nach Anspruch 11 oder Zusammensetzung zur Verwendung nach einem der Ansprüche 1-10, wobei die Zusammensetzung ausgewählt ist aus einem Getränk, einer Kapsel, einer Tablette, einem Pulver, einem Riegel und einem Aufstrich.

## Revendications

1. Composition prébiotique destinée à être utilisée dans un procédé de traitement thérapeutique ou prophylactique de troubles associés à une composition ou à une fonctionnalité perturbée du microbiome intestinal chez un sujet, le trouble étant choisi parmi le surpoids, l'obésité, la résistance à l'insuline et le dysfonctionnement de la barrière intestinale, ladite utilisation comprenant l'administration orale de la composition prébiotique au sujet, ladite composition contenant au moins 0,1 % en poids de matière sèche de polysaccharides de rhamnogalacturonane I (RG-I) provenant de fruits, de carottes, de pois, de chicorée ou de betterave à sucre, lesdits polysaccharides RG-I possédant un poids moléculaire supérieur à 15 kDa et possédant un squelette constitué de résidus d'acide galacturonique et de résidus de rhamnose, lesdits résidus de rhamnose étant contenus dans des résidus d'alpha(1→4)-galacturonique-alpha(1-2)-rhamnose, ledit rapport molaire des résidus d'acide galacturonique aux résidus de rhamnose dans les polysaccharides RG-I étant compris dans la plage de 20:1 à 1:1.

2. Composition prébiotique destinée à être utilisée selon la revendication 1, ladite composition étant ingérée par le sujet pendant une période d'au moins 3 jours en une quantité fournissant au moins 1 mg de polysaccharides RG-I par kg de poids corporel par jour.

3. Composition prébiotique destinée à être utilisée selon l'une des revendications 1 ou 2, dans laquelle les polysaccharides RG-I représentent au moins 20 % en poids des polysaccharides pectiques présents dans la composition prébiotique.

4. Composition prébiotique destinée à être utilisée selon l'une quelconque des revendications précédentes, dans laquelle le rapport molaire des résidus d'acide galacturonique aux résidus de rhamnose dans les polysaccharides RG-I ne dépasse pas 15:1, de préférence ne dépasse pas 12:1, idéalement ne dépasse pas 10:1.

5. Composition prébiotique destinée à être utilisée selon l'une quelconque des revendications précédentes, dans laquelle le rapport molaire des résidus d'arabinose aux résidus de rhamnose dans le polysaccharide RG-I ne dépasse pas 30:1.

6. Composition prébiotique destinée à être utilisée selon l'une quelconque des revendications précédentes, dans laquelle le rapport molaire des résidus de galactose aux résidus de rhamnose dans le polysaccharide RG-I ne dépasse pas de préférence 30:1.

7. Composition prébiotique destinée à être utilisée selon l'une quelconque des revendications précédentes, dans laquelle moins de 85 % des résidus d'acide galacturonique dans les polysaccharides RG-I sont estérifiés sous la forme d'esters méthyliques.

8. Composition prébiotique destinée à être utilisée selon l'une quelconque des revendications précédentes, dans laquelle les polysaccharides RG-I proviennent d'une source végétale choisie parmi la carotte, la pomme, le poivron, les agrumes, la myrtille, le raisin, le pois, la chicorée, la betterave à sucre, l'olive, le gombo et des combinaisons de ceux-ci.

9. Composition prébiotique destinée à être utilisée selon l'une quelconque des revendications précédentes, dans laquelle le sujet souffre ou risque de souffrir d'un trouble métabolique.

10. Composition prébiotique destinée à être utilisée selon l'une quelconque des revendications 1 à 8, dans laquelle le sujet souffre ou risque de souffrir d'un dysfonctionnement de la barrière intestinale.

11. Composition symbiotique comprenant :
• au moins 0,1 % en poids de matière sèche de polysaccharides de rhamnogalacturonane I (RG-I) provenant de carottes ou de pommes, lesdits polysaccharides RG-I possédant un poids moléculaire supérieur à 15 kDa et possédant un squelette constitué de résidus d'acide galacturonique et de résidus de rhamnose, lesdits résidus de rhamnose étant contenus dans des résidus d'alpha(1→4)-galacturonique-alpha(1→2)-rhamnose, ledit rapport molaire des résidus d'acide galacturonique aux résidus de rhamnose dans les polysaccharides RG-I étant compris dans la plage de 20:1 à 1:1 ; et
• 10⁵ à 10¹⁰ ufc/gramme d'*Akkermansia muciniphila* et/ou 10⁶ à 10¹⁰ ufc/gramme de *Bifidobacterium spp.*

12. Composition selon la revendication 11 ou composition destinée à être utilisée selon l'une quelconque des revendications 1 à 10, ladite composition étant choisie parmi une boisson, une capsule, un comprimé, une poudre, une barre et une pâte à tartiner.
